# EUROPEAN PATENT SPECIFICATION

(11) **EP 1 910 536 B1**
(45) Date of publication and mention of the grant of the patent: **09.09.2009**
(21) Application number: 06779850.4
(22) Date of filing: 11.07.2006
(51) Int. Cl.: C12N 15/10, C12Q 1/68

(54) **METHODS FOR IDENTIFYING GENES THAT INCREASE YEAST STRESS TOLERANCE, AND USE OF THESE GENES FOR YEAST STRAIN IMPROVEMENT**
VERFAHREN ZUR IDENTIFIZIERUNG VON DIE STRESSTOLERANZ IN HEFE ERHÖHENDEN GENEN SOWIE VERWENDUNG DIESER GENE ZUR VERBESSERUNG VON HEFESTÄMMEN
PROCÉDÉS D'IDENTIFICATION DE GÈNES QUI AUGMENTENT LA TOLÉRANCE AU STRESS DE LA LEVURE, ET UTILISATION DE CES GÈNES DANS L'AMÉLIORATION DE SOUCHES DE LEVURE

(30) Priority: 26.07.2005 IN DE19772005
(43) Date of publication of application: 16.04.2008
(73) Proprietor: Council of Scientific and Industrial Research, New Delhi 110 011 (IN)
(72) Inventor: PURIA, Rekha Institute of Microbial Technology, Chandigargh 160 036 (IN); CHOPRA, Rohini Institute of Microbial Technology, Chandigargh 160 036 (IN); GANESAN, Kaliannan Inst. of Microbial Technology, Chandigargh 160 036 (IN)
(74) Representative: Loveless, Ian Mark
(86) International application number: PCT/IB2006/001910
(87) International publication number: WO 2007/012934

(56) References cited:
- EP-A- 0 577 915
- EP-A1- 0 645 094
- WO-A-87/03006
- WO-A-94/26933
- WO-A-95/33834
- WO-A-97/01626
- WO-A-99/60138
- WO-A2-20/04090141
- US-B1- 6 528 257
- SHARMA V M ET AL: "Quantitative target display: a method to screen yeast mutants conferring quantitative phenotypes by 'mutant DNA fingerprints'." NUCLEIC ACIDS RESEARCH 1 SEP 2001, vol. 29, no. 17, 1 September 2001 (2001-09-01), pages E86-E86, XP002417901 ISSN: 1362-4962 cited in the application
- RODRIGUEZ-VARGAS S ET AL: "Gene expression analysis of cold and freeze stress in baker's yeast" APPLIED AND ENVIRONMENTAL MICROBIOLOGY, WASHINGTON,DC, US, vol. 68, no. 6, June 2002 (2002-06), pages 3024-3030, XP002304505 ISSN: 0099-2240
- SOBERING ANDREW K ET AL: "Yeast Rpi1 is a putative transcriptional regulator that contributes to preparation for stationary phase." EUKARYOTIC CELL FEB 2002, vol. 1, no. 1, February 2002 (2002-02), pages 56-65, XP002429770 ISSN: 1535-9778
- KIM J H ET AL: "Overexpression of RPI1, a novel inhibitor of the yeast Ras-cyclic AMP pathway, down-regulates normal but not mutationally activated ras function." MOLECULAR AND CELLULAR BIOLOGY AUG 1991, vol. 11, no. 8, August 1991 (1991-08), pages 3894-3904, XP002429771 ISSN: 0270-7306

## Description

### FIELD OF INVENTION

The present invention relates to improvement of yeast for enhanced stress tolerance. More specifically, it relates to identifying genes that enhance the survival of yeast during ethanol production, and use of these genes for improving the performance of yeast strains.

### BACKGROUND OF THE INVENTION

Yeast that can complete ethanol production without much loss in viability is highly desired in distilleries. However, common yeast strains used in distilleries loose viability rapidly, due to high ethanol concentration encountered during fermentation. Besides, yeast also experience higher temperature (particularly in tropical countries) which together with ethanol dramatically reduces viability. Various approaches have been taken to get improved yeast strains with high ethanol and temperature tolerance (thermotolerance). One approach is test yeast isolates from the natural environment for desired properties (Banat et al, 1998). While some of them have high ethanol or temperature tolerance, they may not have all the properties desired, such as higher osmotolerance (i.e., ability to withstand high conc., of solutes such as sugar and salt), faster fermentation rate, and absence of unwanted side products. Another approach is to start with strains which already have several desired properties, and improve them further by mutagenesis and selection for better survival during fermentation (e.g. Ganesan *et al,* 2003, Indian Patent # 189737). A major limitation of this approach is that the improvement is mostly due to mutation in a single gene. Since several genes control stress-tolerance, modifying more than one such gene is expected to provide much higher tolerance than single genes. This will be particularly so for stress tolerance during ethanolic fermentation, since yeast cells encounter more than one kind of stress under these conditions, such as high osmolority, high ethanol concentration, and high temperature. Thus, if genes providing tolerance to these stresses are identified, then they can be rationally engineered to enhance stress tolerance. However, it is not easy to identify these genes by using conventional yeast genetics and molecular biology approaches, for the following reasons.

The conventional approach to identify yeast genes involved in any process is to first identify mutants impaired in that process, categorize them as belonging to different complementation groups (genes) by genetic analysis, and finally identify the genes by using yeast molecular biology and recombinant DNA tools (Kaiser et al, 1994). Mutants may be obtained as spontaneous mutants, or induced by chemical mutagenesis (Kaiser et al, 1994), by transposon insertion mutagenesis (Ross-Macdonald et awl, 1999), or even by introducing ribozyme libraries (Thompson, United States Patent # 6,183,959). Further screening of mutants for desired phenotypes typically involves maintaining a large number of potential yeast mutants as clonal populations (colonies) on the surface of non-selective solid media in petri-plates, and screening them by simultaneously transferring them to solid selective media by replica-plating. The colonies unable to grow on the selective media will be identified, and corresponding colonies will be taken from the non-selective media and further characterized. This process of identifying mutants is referred to as plate-screens. However, this approach is not useful for identifying genes involved in fermentation stress tolerance, since there is no plate screen that can simulate the conditions encountered by yeast within the liquid fermentation broth. Thus, a method was devised in our lab earlier that can simultaneous monitor the fitness of individual mutants of a microbe in mixed populations present in liquid broths (Sharma *et al,* 2001; Sharma & Ganesan, 2003, United States Patent # 6,528,257). By this method several genes with role in fermentation stress tolerance could be identified. Other methods that also facilitate simultaneous monitoring of the fitness of mutants can be used for this purpose. (Brown & Smith, 1997, United States Patent # 5,612,180; Smith *et al,* 1996; Winzeler *et al,* 1999).

However, identifying, genes through mutant phenotypes has certain limitations. Firstly, it is not easy to get mutants impaired in genes that are essential for normal growth and survival. Thus, such genes, if also critical for some other function such as stress tolerance, will be missed. Secondly, many genes are repeated in yeast, i.e., there is more than one gene providing the same function to the organism. Thus, mutating any one of them will not result in a discernable phenotype, and they will be missed in the conventional mutant screens. Moreover, if the purpose of identifying genes involved in a process such as stress tolerance is ultimately to improve the organism, then identifying relevant genes through mutant hunts is not always successful. The reason is though a gene could be important for a biological process by performing an essential step in the process, it may not be performing the rate-limiting step. Thus, overexpressing such a gene will not result in any improvement in the process. Therefore, first identifying all the genes involved in a process, and then overexpressing them one by one to see if they help to improve the process is laborious, time-consuming, and prone to failure. Here we provide an alternate method that overcomes all the above limitations. Besides, our method also overcomes the limitation of lack of plate-screens.

Another approach to assign function to genes is expression profiling using microarrays. By expression profiling, the expression levels of almost all the genes of an organism are simultaneously determined (e.g., Hughes et al., 2000; Wu et al, 2001; Fabrizio et al, 2005; Vrana et al., 2003). If a set of genes are expressed higher under one condition compared to another, then it is assumed that these genes have some role to play under the first condition. However, this assumption is not supported by studies where attempts were made to correlate expression of genes with their role under a particular environmental condition (Giaever et al., 2002; Birrell et al., 2002). The correlation found was hardly better than what can be seen by chance, and thus assigning gene function based on expression levels can be misleading. In contrast, mutation based methods (cited above) that assign function based on mutant phenotypes are much more reliable in providing biological role to genes. Similarly, methods (discussed below) that are based on deliberate overexpression of genes are also reliable, since they also provide biological role to genes on the basis of phenotype of the organism.

The present invention involves simultaneous screening for genes that upon overexpression enhance the stress tolerance of yeast. This is in contrast with known methods that overexpress one gene at a time to enhance stress tolerance; e.g., overexpression of HAL1, YAK1, SOD1, SOD2 and TPS1 individually have been shown to increase stress tolerance to various stress conditions (Chen *et al,*1995; Davidson *et al,*1996*;* Gaxiola *et al,* 1992*;* Hartley *et al,*1994*;* Soto *et al,*1999*).* In many other cases yeast strains have been engineered using overexpression strategy so that they efficiently ferment substrates like starch, cellobiose, lactose, xylose etc. (Adam et *al,* 1995; Muslin *et al,* 2000; Walfridson *et al,* 1995). Overexpression of GPD1 has been shown to increase glycerol production by 1.5-2.5 fold (Remize *et al,* 1999). We have devised our method to circumvent the limitation of a lack of a plate screen for fermentation stress tolerance, and also lack of much understanding about the genes involved in this process..Instead of first identifying genes involved in stress tolerance, and then overexpressing them one by one to see if they improve stress tolerance, in our method a novel approach is taken to directly identify genes that enhance stress tolerance upon overexpression. Genome-scale overexpression screens have been carried out by others, e.g., to identify lethal or impaired growth phenotypes ( Espinet et *al,* 1995; Boyer *et al,* 2004), and to identify previously uncharacterized cell cycle genes (Stevenson *et al,* 2001). All these screens took advantage of easy plate screens to identify desired properties of the organism. In contrast, in our method, genes conferring enhanced stress tolerance are identified from a mixed pool of large number of yeast transformants overexpressing different genes. This is particularly advantageous for identifying genes conferring phenotypes for which there is no plate screen, such as fermentative stress tolerance.

US 6 528 257 discloses a method for the simultaneous monitoring of abundance of individual mutants in a microbe in mixed populations.

W094126933 discloses a method of genetic footprinting.

Sharma et al, Nucleic Acids Research, vol. 29, No.17, p E86 describes screening of yeast mutants.

WO95/33834 discloses a method of increasing stress tolerance.

WO2004/090141 discloses a screening method.

### OVERVIEW OF THE INVENTION

The present invention involves the development of a method for simultaneous identification of genes conferring desired phenotypes by screening a mixed population of yeast transformants. A library of plasmids bearing different genes with their respective promoters or under the control a strong promoter is transformed into yeast. This library should be large enough to carry almost all the genes of an organism with high probability. The pool of yeast transformants is then subjected to selection, e.g., for better survival under fermentation conditions. The cells that survive one round of selection are again subjected to another round of selection. In one approach, selection is repeated about six times. At the end the pool of survivors is expected to have mostly those transformants that can survive the selection conditions much better than the starting pool of transformants, which can be confirmed by a comparing the performance of these two pools. To ensure that the enhanced survival is due to the genes carried on plasmids, and not due to any mutation in the genome of the organism, the plasmids are recovered from yeast, retransformed into wild-type yeast and the phenotype confirmed. The genes carried on these plasmids are then identified by methods such as DNA sequencing. These genes are then studied one by one to confirm their role in stress tolerance. The expression of these genes can be modulated in yeast one at a time, or in combination, to enhance the performance of yeast during fermentation. In another approach the pool of yeast transformants is subjected to selection for only a few rounds of selection. At the end of this selection this pool will be enriched with those that are able to survive better than the average population, but the survival of most of the transformants will be similar to that of starting pool of transformants. To identify the genes carried by the better survivors the following steps are followed. Total DNA is isolated from the starting population of transformants and from the selected population. The insert DNA carried on plasmids from the total DNA is selectively amplified of by using plasmid-specific primers. The amplified insert DNA fragments of the starting population of transformants are labeled with one fluorescent dye, and that of the selected population with another fluorescent dye. The labeled DNA probes are then mixed and hybridized to a microarray spotted with DNA corresponding to almost all the genes of yeast. The DNA spots on the microarray that show enhanced signal for probe corresponding to the selected population compared to that of starting population are then identified. The genes that correspond to these DNA spots are then shortlisted as those that increase the stress tolerance of yeast upon overexpression. The role of these genes may be further confirmed by additional experiments involving individual overexpression or deletion of these genes.

### DETAILED DESCRIPTION OF THE INVENTION

Accordingly, the present invention provides a method to identify genes that upon overexpression enhance the stress tolerance of yeast, which comprises,
- transforming yeast with a library of yeast genes cloned in a plasmid to provide a large number of yeast transformants.
- pooling the transformants to provide a starting population of transformants.
- subjecting the population of transformants to selection under liquid conditions such that the viability of the population of cells decrease 3 to 200-fold, thereby enriching those transformed cells that can survive better under these conditions,
- recovering surviving cells and growing them in a defined minimal medium that allows growth of only those cells retaining plasmids,
- subjecting these cells to additional rounds of selection by repeating steps c and d,
- recovering cells that have survived at least three rounds of selection,
- comparing the selected population of cells to the starting population of cells under selection conditions to confirm the enhanced survival of selected cells,
- plating out the selected pool of cells to get isolated colonies,
- isolating DNA from individual yeast colonies and transforming into E. coli to recover the plasmid present in individual yeast colonies,
- transforming these plasmids into yeast and testing the transformants to check if the plasmids really confer enhanced tolerance under the selection conditions, and,
- identifying the genes carried on the plasmids, which confer enhanced stress tolerance, by sequencing.

In one embodiment of the invention, genes that contribute to enhanced fitness during selection are directly identified using microarray hybridization, which comprises,
- transforming yeast with a library of yeast genes cloned in a plasmid to provide as large number of yeast transformants.
- pooling the transformants to provide a starting population of transformants.
- subjecting the population of transformants to selection under liquid conditions such that the viability of the population of cells decrease 3 to 200-fold, thereby enriching those transformed cells that can survive better under these conditions,
- recovering surviving cells and growing them in a defined minimal medium that allows growth of only those cells retaining plasmids,
- subjecting these cells to additional rounds of selection by repeating steps c and d,
- recovering cells that have survived at least one round of selection, '
- isolating total DNA from the starting population of transformants and the selected population,
- amplifying specifically only the insert DNA carried on plasmids from the total DNA by using plasmid-specific primers,
- labeling of insert DNA fragments of the starting population of transformants with one fluorescent dye, and that of the selected population with another fluorescent dye.
- mixing and hybridizing the labeled probes to a microarray spotted with DNA corresponding to almost all the genes of yeast.
- identifying the DNA spots on the microarray that show enhanced signal for probe corresponding to the selected population compared to that of starting population, and,
- identifying genes that correspond to these DNA spots as those which increase the stress tolerance of yeast during selection.

In another embodiment of the invention, yeast is transformed with a library of genes from organisms that are already tolerant to the particular stress.

In yet another embodiment of the invention, plasmids carrying genes highly enriched during selection can be directly isolated from library by colony hybridization.

In yet another embodiment of the invention, the plasmid is an expression plasmid with a constitutive promoter.

In yet another embodiment of the invention, the plasmid is an expression plasmid with an inducible promoter.

The stress may be that encountered by yeast under alcohol producing conditions, particularly at high temperature.

Glucose may be used as a raw material for alcohol production.

Sucrose or molasses or any other complex carbon source may be used as raw material for alcohol production.

The organism may be a laboratory strain of yeast. The organism may be an industrial strain of yeast.

The organism may be any microorganism that needs to be improved for stress tolerance.

The stress may be any adverse condition encountered by microorganisms in industries.

The process of present invention is illustrated in the examples given below, which should not be however construed to limit the scope of present invention.

### EXAMPLE 1

**Screening of yeast genomic expression library for genes that enhance stress tolerance.** Yeast genomic DNA library made under the control of a constitutive ADH1 (Alcohol dehydrogenasel) promoter, in a centromeric plasmid with URSA3 as a selection marker was obtained from American type Culture collection (ATCC). Yeast strain FY3 (Winston et al., 1995) obtained from Fred Winston (Department of Genetics, Harvard Medical School, Boston, MA 02115, USA) was used for all transformations. The plasmids from this library were transformed into strain FY3 by standard transformation protocol (Kaiser *et al,* 1994). Transformants (~10⁵) were pooled together and subjected to 5 rounds of fermentation at 38°C. In parallel same pool was subjected to 6 rounds of fermentation at 30°C. The time of harvest and transfer to the successive rounds was aimed such that there was about 90% killing of cells. Plating on the minimal media at the end of each round revealed that about 50% of the cells have lost their plasmid. After repeated rounds of fermentation both the populations were treated separately.

**Results from 38°C selection:** From the selected population plasmids were taken out by standard protocols and retransformed into *E.coli* JM109. Plasmids were purified from the transformants by standard methods (Sambrook *et al,* 1989) and digested with restriction enzyme Xho1. Plasmids showing similar digestion pattern were later digested with set of two or three enzymes (XhoI, XbaI and EcoRV). 25 plasmids showing unique pattern of digestion were then transformed into yeast and their fermentation studies were done at 38°C with control strain transformed with vector alone. Out of the 25 plasmids 10 showed 20-1000 fold-increased viability after 49-52 hrs of fermentation, producing slightly more or same amount of the alcohol as compared to control. Sequencing done with an ADH1 promoter specific primer and BLAST sequence similarity search (Altshul *et al,* 1997) showed that all nine inserts have complete RPI1 gene including its promoter. Besides, the RPI1 gene was present in both orientations with respect to ADH1 promoter. From this it could be inferred that introducing an additional copy of RPI1 expressed from its own promoter is sufficient to provide enhanced stress tolerance during fermentation.

**Results from 30°C selection:** The population obtained after 6 rounds of repeated selection was compared with unselected parent (starting) population for viability and rate of ethanol production. While the rate of ethanol production was comparable, the selected population showed about 150 fold better survival than the parent population after 127 hrs of fermentation. To rule out the possibility of contribution of any of enriched spontaneous mutation in enhanced viability, plasmids were retrieved from selected population and transformed into *E. coli* JM109. Plasmids isolated from a random pool of 1000 *E. coli* colonies were transformed back into yeast strain FY3 by following standard protocols. Approximately 20,000 yeast transformants were pooled together and subjected to an additional round of fermentation at 30°C. These transformants showed much better viability than the parent strain, confirming that the genes carried on plasmids confer enhanced stress tolerance. These plasmids were again transferred to *E. coli,* and plasmids from 38 colonies were isolated and analyzed by restriction digestion to identify unique clones. Fifteen unique plasmids were transformed back into FY3 and compared in 30°C fermentation for relative survival and ethanol production with respect to the control population. Among these, nine appeared promising. The genes carried by these plasmids were identified by sequenced using ADH1 specific primer and BLAST sequence similarity search. Out of the nine clones, five corresponded to WSC4, three carried the uncharacterized gene YL055C- and one carried an insert of about 2.9-Kb encompassing the whole of . Carp2, most of SRA1 and a small portion of CKA1. Further studies were done to confirm the role of these genes in fermentation.

### EXAMPLE 2

**Characterization of RPI1.** From preliminary studies, based on sequencing, we inferred that addition of a single copy of RPI1 (Ras cAMP pathway inhibitor 1) is sufficient to provide enhanced stress tolerance during fermentation. Hence we subcloned the entire 2.6kb insert isolated from one of the RPI1 clones at the XhoI site of an integrative vector pRS306. This vector was linearized within the URA3 gene of the vector and transformed into yeast strain FY3 for targeted integration at the URA3 locus of the genome. Transformants were selected by uracil prototrophy on minimal media plates. An additional copy of RPI1 was thus integrated in the genome. Earlier studies have shown that RPI1 disruptants are not lethal (Kim *et al,* 1991). RPI1 disruptants were made by insertional mutagenesis using Tn3. Disruptants were confirmed by sequencing using Tn3 specific primers. These overexpression and disruption strains were then used for fermentation studies done at 38°C and 30°C. Fermentation studies at 38°C were done with 20% glucose for 38-44 hrs while fermentation studies at 30°C were done with 25% glucose for 108-114 hrs. Viability was monitored at the end of fermentation. Strains with additional copy of RPI1 showed many-fold enhanced survival compared to control population at both 38°C and 30°C fermentation studies. While the strains with disrupted copy of RPI1 showed considerably reduced survival confirming the role of RPI1 in stress tolerance. But the strains with additional copy of RPI1 showed slow rate of fermentation in the beginning though there was no difference in the amount of ethanol finally made with respect to wild-type strain.

Mixed culture studies: It could be argued that the enhanced survival seen in RPI1 overexpression strain is due to slow rate of fermentation. To rule out this possibility mixed culture fermentations were done. Strains with additional copy of RPI1 were mixed in equal proportion with control wild-type population having G418 resistance marker. The G418 resistance marker does not affect performance or viability of cells during fermentation, but serves as a marker to distinguish the control strain from the test strain after mixed fermentation. The fermentation rate of mixed culture was comparable to that of wild-type strain. After fermentation, the test strain with additional copy of RPI1 showed about hundred fold better survival than wild type at both 38°C (Table la) and 30°C (Table 1b). In contrast, the viability of strain deleted for RPI1 was extremely poor compared to the control strain. These results show that RPI1 is critical for stress tolerance of yeast during ethanolic fermentation.

**Table 1a**

| **Effect of RPI1 gene expression level on survival of yeast after 37 hr of fermentation at 38°C** | |
|---|---|
| **Strain name* (gene copy number)** | **Relative survival of G418^{S} test strain as a percent of total population** |
| Control (1) + G418^{R} Control (1) | 64.0% |
| RPI1↑ (2) + G418^{R} Control (1) | 99.98% |
| RPI1Δ (0) + G418^{R} Control (1) | 1.66% |

| | |
|---|---|
| ↑ = Gene overexpressed Δ = Gene deleted | |

**Table 1b**

| **Effect of RPI1 gene expression level on survival of yeast after 114 hr of fermentation at 30°C** | |
|---|---|
| **Strain name (gene copy number)** | **Relative survival of G418^{S} test strain as a percent of total population** |
| Control (1) + G418^{R} Control (1) | 43 % |
| RPI1↑ (2) + G418^{R} Control (1). | 100 % |
| RPI1Δ (0) + G418^{R} Control (1) | 0 % |

RAI1 was earlier identified as a high copy suppressor of Ras2 mutation suppressing the heat shock phenotype induced by Ras2 mutation (Kim *et al,* 1991). Later studies proved that it is not associated with Ras-cAMP pathway, but possibly a transcription factor that prepares cells for entry into stationary phase (Sobering *et al,* 2002). Our studies show that RPI1 is critical for stress tolerance during ethanolic fermentation as well, and its overexpression can be used to enhance the survival of yeast many-fold.

### EXAMPLE 3

**Characterization of WSC4.** WSC4 (cell Wall integrity and Stress response Component) gene, identified from 30°C fermentation, is known to be required for secretary protein translocation, for maintenance of cell wall integrity and for stress response in *S.cerevisiae* (Verna *et al,* 1997). To study its behavior in fermentation stress the complete WSC4 gene along with its promoter was subcloned in integrative vector PRS306. This gene was then integrated in genome by homologous recombination at ura3 locus, thus increasing copy number by one. WSC4 disruptants were also made by transposon mutagenesis and further confirmed by sequencing. Fermentation studies done at 30°C with 25% glucose for 108 hr showed that it could enhance survival 5-10 fold compared to wild type. Further, mixed culture fermentation studies done along with wild type strain showed that WSC4 does improve stress tolerance of *S*. *cerevisiae* strain (Table 2). Abundance of WSC4 deletion strains is considerably reduced in mixed pool. This study shows that WSC4 can be exploited to improve upon stress tolerance during fermentation.

**Table 2**

| **Effect of WSC4 gene expression level on survival of yeast after 109 hr of fermentation at 30°C** | |
|---|---|
| **Strain name (gene copy number)** | **Relative survival of G418^{S} test strain as a percent of total population** |
| Control (1) + G418^{R} Control (1) | 44% |
| WSC4↑ (2) + G418^{R} Control (1) | 73% |
| WSC4Δ (0) + G418^{R} Control (1) | 6.1% |

### EXAMPLE 4

**Characterization of YIL055c.** YIL055c; a gene with an unknown function, was also isolated from the overexpression screen at 30°C. To confirm its role in stress tolerance the ORF of this genes was subcloned under the control of strong GPD1 promoter in the integrative vector pGV8. The recombinant construct was then integrated at the ura3 locus of strain FY3. This gene was also disrupted by transposon mutagenesis and confirmed by sequencing. Fermentation studies done at 30°C showed that upon overexpression of YIL055c the rate of fermentation is considerably reduced, though it can complete the fermentation like wild type. Disruptants showed normal fermentation rate. In mixed culture fermentation done at 30°C, these overexpression strains fermented at equal rate as wild type mixed culture control. These stains still conferred enhanced survival compared to wild-type cells (Table 3). This indicates that enhanced survival is not due to reduced rate of fermentation. The role of YIL055c in stress tolerance is quite similar to that of RPI1, and thus these genes are likely to be part of the same stress response pathway.

**Table 3**

| **Effect of YIL055C expression level on survival of yeast after 108 hr of fermentation at 30°C** | |
|---|---|
| **Strain name (gene copy number / overexpression under GPD promoter)** | **Relative survival of G418^{S} test strain as a percent of total population** |
| Control (1) + G418^{R} Control (1) | 47% |
| YIL,055C↑ (GPD) + G418^{R} Control (1) | 94% |
| YIL055CΔ (0) + G418^{R} Control (1) | 17.5% |

### EXAMPLE 5

**Characterization of SRA1.** Another clone screened encompassed three genes including a fragment of SRA1. SRA1 part from this clone was subcloned under the control of strong promoter GPD1 in pGV8 and integrated in the yeast genome. Fermentation studies were done with SRA1 overexpression clone at 30°C. It showed several fold enhanced survival after 108 hrs of fermentation with 25% sugar. Strains with overexpressed SRA1 showed initial lag in ethanol production, but completed the fermentation in equal time span as taken by the wild type. Further mixed culture fermentation studies showed normal fermentation rate, but enhanced survival compared to the control strain (Table 4). SRA1 disruptants showed reduced survival during normal growth itself and thus no fermentation studies were done with these strains.

**Table 4**

| **Effect of SRA1 expression level on survival of yeast after 108 hr of fermentation at 30°C** | |
|---|---|
| **Strain name (gene copy number / overexpression under GPD promoter)** | **Relative survival of G418^{S} test strain as a percent of total population** |
| Control (1) + G418^{R} Control (1) | 47% |
| SRA1↑ (GPD) + G418^{R} Control (1) | 94% |

SRA1 has cAMP-dependent protein kinase inhibitor activity. Overexpression of SRA1 shifts equilibrium of association or dissociation of PKA into its sub units towards undissociated state (Portela *et al,* 2001). It also leads to hyperaccumulation of glycogen and improved heat shock resistance. Thus, SRA1 upon overexpression leads to lower PKA activity and enhanced stress tolerance. Reduced PKA activity results in decreased growth rate (Van Dijck *et al,* 2000). This could explain reduced rate of fermentation observed upon overexpression of SRA1. But its enhanced viability in mixed culture fermentation shows that the enhanced stress tolerance is not due to reduced rate of fermentation. Isolation of SRA1 by overexpression strategy proves the validity of the strategy, as role of cAMP pathway in stress tolerance is already established through studies with RAS mutants.

### EXAMPLE 6

**Genome scale fitness profiling of overexpression strains.** Microarrays are widely exploited to monitor whole genome in single chip. It gives a better picture of the interactions among thousands of genes simultaneously. Here we used DNA based microarray, to simultaneously monitor the genes present on plasmids, for their role in fermentation stress. Yeast strain FY3 was transformed with a whole genome overexpression library, as described in Example 1. A pool of about 20,000 transformants was grown in a minimal medium for 24hr, and divided into two pools. One pool was kept as unselected population while other was subjected to two rounds of fermentation at 38°C. After each round cells were harvested and grown in a minimal medium to enrich for cells retaining plasmids. At the end of two rounds total DNA was isolated from selected and unselected pool of cells by standard protocols (Kaiser *et al,* 1994). Insert DNA fragments of plasmids present in the total DNA were specifically amplified using a primer 5'-CTCGAGCTACGTCAGGG-3') complementary to adapter sequences present on both sides of the insert DNA. A PCR reaction was set up in 25µl volume. It contained 100 ng template, 1X Taq buffer (NEB), 0.4mM primer, 0.2mM dNTPs and 5U Taq DNA polymerase. Thermal cycling conditions were: denaturation at 95°C for 5min followed by 30 cycles of denaturation at 94°C for 1min, annealing at 60°C for 1min and extension at 72°C for 4 min. This was followed by a final extension at 72°C for 10min. The PCR products were checked for uniformity in the size range in both populations by agarose gel electrophoresis and then purified using PCR purification kit (Qiagen). One µg of purified PCR product from each population was labeled either with Cy3 or Cy5 by random primer labeling. In each labeling reaction 1µg of pure PCR product, 10 µg of random primers (nonamer), 2 ng Arabidopsis control DNA, 0.5mM dNTPs without dCTP, 1X klenow buffer, 2 µl of either fluorophor (Cy5/Cy3 dCTP) and 50U Klenow fragment of DNA polymerase (NEB) were added. Reaction was incubated at 37C for 12 hr and terminated by adding 2.5 µlof 0.5M EDTA. The labeled product was purified using PCR purification kit. Labeling was quantitated by comparing serial dilution of impure sample with pure sample spotted on glass slide. These were scanned on microarray to find out the percent incorporation. For hybridization Cy5 and Cy3 labeled DNA fragments were mixed together in equal ratios and the volume was made up to 18ul. This was heat denatured at 95°C for 2min. Hybridization reaction of 80 µl was set up. In this 18 µl of labeled probe, 1X hybridization buffer, 1.6 µl 10%SDS and 40 µl formamide was mixed. This whole volume is put on microarray glass slide and carefully covered with a coverslip. Hybridization was set up in hybridization chamber and incubated in water bath at 42°C for 12hrs.Washing of slides were done by following standard protocols (Bowtell *et al,* 2002). These slides were then scanned on Axon 4000B microarray scanner. Genepix Pro software was used for analysis of data. Experiments were done with two separate libraries to check consistency. Besides, dye-swap experiments were also done to rule out bias in labeling. Features (genes) that show higher signal for DNA of selected population compared to starting population are those which have become enriched during selection, and thus, have higher fitness during selection conditions.

### EXAMPLE 7

**Colony hybridization to recover enriched genes conferring enhanced fitness.** Genes conferring role in stress tolerance can directly be isolated from genomic library using colony hybridization. Whole gene probes were prepared for the genes consistently enriched in all microarrays. Plasmids retrieved from the selected population at the end of four rounds of fermentation were transformed into *E.coli* by standard protocol. 1000 *E.coli* transformants were patched on LB-amp plates. For colony hybridization standard protocol using Hybond (N) nylone membrane was followed (Sambrook *et al,* 1989). For each gene many clones could be isolated. Plasmids (from randomly picked clones) were isolated by alkaline lysis method, and restriction digested with XhoI enzyme to identify inserts of unique size. Those appearing unique were sequenced using ADHI specific primer. These were further characterized and transformed into yeast to confirm their role in fermentation stress tolerance. Some of the inserts carried more than one gene or additional partial genes. Besides sequencing helped in finding the orientation of the genes with respect to ADHI promoter. Inserts corresponding to RPI1, MKT1, ECM39, SOL1 and ADE16 were identified and characterized using this approach.

### EXAMPLE 8

**Confirmation of role of WSC2.** Microarray based screening showed WSC2 to be highly enriched in all sets of experiments. Thus, its overexpression and disruption clones were made to confirm its role. Overexpression clones were obtained by replacing its endogenous promoter with GPD1 promoter (Petracek *et al,* 2002) while disruptants were made by substituting WSC2 coding region with URA3 marker. These were transformed into URA3 deletion strain. Fermentation studies done at 38°C with 20% sugar showed that WSC2 overexpression clones survive 50-100 fold better than wild type at the end of fermentation. It does not show any change in rate of fermentation compared to wild-type. Even in mixed culture fermentation studies done at 38°C, this strain became the predominant one at the end of fermentation compared to the G418^{r} control strain (Table 5). WSC2 belongs to the same family as WSC4 and is involved in cell wall integrity pathway. WSC genes are functionally redundant. One can not identify their role until all are deleted or deleted in combination. However this overexpression strategy could identify the role of individual members of redundant genes. WSC is a family of signalling molecules, which activate downstream Rho1 and MAP kinase pathways important in environmental stress response. Moreover, there is no obvious effect on growth or fermentation rate suggesting that these act in PKA independent manner. Thus, further exploration of WSC pathway genes can help in improving strains for fermentation stress tolerance.

**Table 5**

| **Effect of WSC2 expression level on survival of yeast after 38 hr of fermentation at 38°C** | |
|---|---|
| **Strain name (gene copy number / overexpression under GPD promoter)** | **Relative survival of G418^{S} test strain as a percent of total population** |
| Control (1) + G418^{R} Control (1) | 45.7% |
| WSC2↑ (GPD) + G418^{R} Control (1) | 97% |

### EXAMPLE 9

**Other genes identified by fitness profiling.** Many other genes that showed up consistently in microarray were confirmed for their role in fermentation stress tolerance by following mixed culture fermentation studies. Many plasmids isolated from second round selected population, identified by colony hybridization for specific genes, were transformed into FY3 yeast strain. These transformants were subjected to mixed culture fermentation studies. Here overexpression test strains were mixed with G418^{R} wild-type control strain in equal proportion. Fermentation studies were carried out at 38°C with 20% sugar. G418 sensitive cells surviving at the end of fermentation were compared with G418^{R} cells. In case of ECM39 (Table 6) and MKT1 (Table 7) overexpression clones more than 90% population was dominated by G418 sensitive test strains, while in case of ADE16 more than 85% population corresponded to ADE16 overexpressing strain (Table 8). SOL1 transformants dominated mixed culture population by 80% (Table 9). ECM39, MKT1 and ADE16 genes were present in opposite orientation with respect to ADH1 promoter in the plasmids. It appears that for these genes addition of a single copy is sufficient to confer enhanced stress tolerance during fermentation. Though all these genes are known in the literature, only our approach has brought out their role in fermentative stress tolerance.

**Table 6**

| **Effect of ECM39 expression level on survival of yeast after 39 hr of fermentation at 38°C** | |
|---|---|
| **Strain name (gene copy number / overexpression under GPD promoter)** | **Relative survival of G418^{S} test strain as a percent of total population** |
| Control (1) + G418^{R} Control (1) | 50% |
| ECM39↑ (?) + G418^{R} Control (1) | 93% |

**Table 7**

| **affect of MKT1 expression level on survival of yeast after 38 hr of fermentation at 38°C** | |
|---|---|
| **Strain name (gene copy number / overexpression under GPD promoter)** | **Relative survival of G418^{S} test strain as a percent of total population** |
| Control + G418^{R} Control (1) | 48.27% |
| MKT1↑+ G418^{R} Control (1) | 92.5% |

**Table 8**

| **Effect of ADE16 expression level on survival of yeast after 38 hr of fermentation at 38°C** | |
|---|---|
| **Strain name (gene copy number / overexpression under GPD promoter)** | **Relative survival of G418^{S} test strain as a percent of total population** |
| Control (1) + G418^{R} Control (1) | 48.27% |
| ADE16↑ (?)+ G418^{R} Control (1) | 84.2% |

**Table 9**

| **Effect of SOL1 expression level on survival of yeast after 38 hr of fermentation at 38°C.** | |
|---|---|
| **Strain name (gene copy number / overexpression under GPD promoter)** | **Relative survival of G418^{S} test strain as a percent of total population** |
| Control + G418^{R} Control (1) | 44.44% |
| SOL1↑ + G418^{R} Control (1) | 86.4% |

**To summarize, the present invention has several advantages:**
- In the present invention almost all the genes of an organism that can possibly **enhance stress tolerance upon overexpression are simultaneously identified.** This is in contrast with known methods where genes involved in stress tolerance are first identified and then overexpressed one at a time to check if they improve stress tolerance. The limitation of the current methods is that only some of the overexpressed genes will result in improvement. The reason is that only some of the genes involved in a process will be rate limiting, and only if these genes are overexpressed there will be any improvement. On the other hand, in our approach only those genes that actually improve stress tolerance are directly identified. Thus, **our invention is less laborious and more efficient.**
- The present invention will **also identify genes that are essential for growth as well as stress tolerance.** In contrast, the mutation-based approaches will mostly miss such genes, since mutants impaired in the essential genes will not be able to grow in the first place for them to be further studied.
- The present invention **will also identify members of duplicated genes.** In contrast; the mutation-based approaches will frequently miss such genes since the duplicated genes often compensate for each other's loss of function.
- The present invention **also overcomes the limitation of lack of plate-screens for studying certain phenotypes such as stress tolerance during ethanolic fermentation in liquid broths.** This is possible since it allows the abundance of all the transformants present in a single pool to be quantitatively followed, by actually monitoring the abundance of the genes carried on plasmids present in these transformants. That is, the genes carried by the plasmids themselves serve as DNA tags to differentiate different transformants present in the pool.
- The present invention also allows small **fitness contributions of different genes to be quantitatively studied.** This is possible since all the transformants are studied in a single pool and thus experience identical and competitive selection conditions.

### REFERENCES

### U.S. Patent Documents

1. Brown P & Smith V (1997) "Genetic footprinting: insertional mutagenesis and genetic selection" United States Patent # 5,612,180
2. Sharma VM & Ganesan K (2003) "Method for the simultaneous monitoring of individual mutants in mixed populations" United States Patent # 6,528,257.
3. Thompson JD (2001) "Method for target site selection and discovery" United States Patent # 6,183,959..

### Other Publications

1. Adam et al, "Induced expression of bacterial betaglucosidase activity in Saccharomyces", Yeast, vol 11,pp. 395-406 (1995).
2. Altshul et al, "Gapped BLAST and PSI-BLAST: a new generation of protein database search programs", Nucleic Acids Res, vol. 25, pp. 3389-3402 (1997).
3. Banat et al, "Review: Ethanol production at elevated temperatures and alcohol concentrations: Part I - Yeasts in general", World Journal of Microbiology and Biotechnology vol 14, pp. 809-821 (1998).
4. Birrell et al., "Transcriptional response of Saccharomyces cerevisiae to DNA-damaging agents does not identify the genes that protect against these agents", Proc Natl Acad Sci U S A. vol 99 pp. 8778-.83 (2002).
5. Bowtell et al, DNA Microarrays. A molecular cloning manual. Cold spring harbor laboratory press. Cold spring harbor, New york. (2002).
6. Boyer et al, "Large-scale exploration of growth inhibition caused by overexpression of 7. genomic fragments in Saccharomyces cerevisiae", Genome Biol. vol 5(9):R72,. Epub (2004).
8. Chen et al, "Consequences of the overexpression of ubiquitin in yeast: elevated tolerances to osmostress, ethanol and canavanine, yet reduced tolerances of cadmium, arsenite and parmomycin", Biochim Biophys Acta, vol 1268, pp.59-64 (1995).
9. Davidson et al, " Oxidative stress is involved in heat-induced cell death in saccharomyces cerevisiae", Proc Natl Acad USA, vol 93, pp. 5116-5121 (1996).
10. Espinet et al, "An efficient method to isolate yeast genes causing overexpression-mediated growth arrest", Yeast, vol 11, pp. 25-32 (1995).
11. Fabrizio et al, "Analysis of gene expression profile in yeast aging chronologically", Mech Ageing Dev. vol 126, pp. 11-6 (2005).
12. Ganesan et al, "An improved process for the production of alcohol using improved thermotolerant flocculent strains of yeast Saccharomyces" Indian Patent # 189737 (2003).
13. Gaxiola et al, " A novel and conserved salt-induced protein is an important determinant of salt tolerance in yeast", EMBO J, vol 11, pp. 3157-3164 (1992).
14. Giaever et al, "Functional profiling of the Saccharomyces cerevisiae genome" Nature, vol 418, pp. 387-91 (2002).
15. Hartley et al, " The YAK1 protein kinase of Saccharomyces cerevisiae moderates thermotolerance and inhibits growth by an Sch9 protein kinase independent mechanism", Genetics, vol 136, pp. 465-474 (1994).
16. Hughes et al, "Functional discovery via a compendium of expression profiles", Cell, vol. 102, pp. 109-26 (2000).
17. Kaiser et al, Methods in yeast genetics. A Cold Spring Harbor Laboratory manual. Cold Spring Harbor laboratory press, New york (1994).
18. Kim et al, "Overexpression of RPI1, a novel inhibitor of the yeast Ras-cyclic AMP pathway down-regulates normal but not mutationally activated ras function", Mol Cell Biol, vol. 11, pp.3894-904 (1991).
19. Liu "Constructing yeast libraries", Methods Enzymol, vol. 350, pp.72-86 (2002).
20. Muslin et al, " Overexpression, purification, and characterization of a barley alphaglucosidase secreted by Pichia pastoris", Prot expr purif, vol 18, pp. 20-26 (2000).
21. Petracek et al, " PCR-based engineering of yeast genome", Methods Enzymol, vol. 350, pp 445-468 (2002).
22. Portela et al, " Evaluation of in vivo activation of protein kinase A under non-dissociable conditions through the overexpression of wild-type and mutant regulatory subunits in Saccharomyces cerevisiae", Microbiology, vol.147, pp.1149-1159 (2001).
23. Remize et al, "Glycerol overproduction by engineered Saccharomyces cerevisiae wine yeast strains lead to substantial changes in By-product formation and to a stimulation of fermentation rate in stationary phase", Appl Environ Microbiol, vol 65, pp.143-149 (1999).
24. Ross-Macdonald et al, "Large-scale analysis of the yeast genome by transposon tagging and gene disruption", Nature, vol 402, pp. 413-8 (1999).
25. Sambrook et al, Molecular cloning. A laboratory manual. Cold Spring Harbor Laboratory press. New York. (1989).
26. Sharma et al, "Quantitative target Display: a method to screen yeast mutants conferring quantitative phenotypes by 'mutant DNA fingerprints', Nucleic Acids Res, vol 29, pp. E86 (2001).
27. Smith et al, "Functional analysis of the genes of yeast chromosome V by genetic footprinting", Science, vol 274, pp.2069-2074 (1996).
28. Sobering et al, "Yeast Rpil is a putative transcriptional regulator that contributes to preparation for stationary phase", Eukaryot Cell, vol. 1, pp.56-65 (2002).
29. Soto et al, " Accumulation of trehalose by overexpression of tps1, coding for trehalose-6-phosphate synthase, causes increased resistance to multiple stresses in the fission yeast schizosaccharomyces pombe", Appl Environ Microbiol, vol 65, pp. 2020-2024 (1999).
30. Stevenson et al, "A large-scale overexpression screen in Saccharomyces cerevisiae identifies previously uncharacterized cell cycle genes", Proc Natl Acad Sci U S A, vol 98, pp. 3946-51 (2001).
31. Van Dijck et al, "A baker's yeast mutant (fill) with a specific, partially inactivating mutation in adenylate cyclase maintains a high stress resistance during active fermentation and growth", J Mol Microbiol Biotechnol., vol.2, pp.521-530 (2000).
32. Vema et al, "A family of genes required for maintenance of cell wall integrity and for the stress response in Saccharomyces cerevisiae", Proc Natl Acad Sci U S A., vol. 94, pp.13804-9 (1997).
33. Vrana et al, "Use of microarray technologies in toxicology research", Neurotoxicology vol 24, pp. 321-32 (2003).
34. Walfridsson et al, "Xylose metabolizing Saccharomyces strains overexpressing the TKL1 and TALI genes encoding pentose phosphate pathway enzymes transketolase and transaldolase", Appl Environ Microbiol, vol 61, pp. 4184-4190 (1995).
35. Winston et al, "Construction of a set of convenient Saccharomyces cerevisiae strains that are isogenic to S288C", Yeast, vol 11, pp. 53-55 (1995).
36. Winzeler et at, Functional characterization of the S. cerevisiae genome by gene deletion and parallel analysis", Science, vol 285, pp. 901-906 (1999).
37. Wu et al, "Plant gene expression profiling with DNA microarrays", Plant Physiology and Biochemistry, vol 39, pp. 917-926 (2001).

## Claims

1. A method for identifying genes for, increasing the stress tolerance of yeast wherein the said method comprising of:
a. transforming yeast with a library of yeast genes cloned in a plasmid to provide a large number of yeast transformants.
b. pooling the transformants obtained in step (a) to provide a starting population of transformants.
c. subjecting the population of transformants obtained in step (b) to selection under liquid conditions such that the viability of the population of cells decrease 3 to 200-fold, thereby enriching those transformed cells that can survive better under these conditions,
d. recovering surviving cells obtained in step (c) and growing them in a medium that allows growth of only those cells retaining plasmids,
e. subjecting the cells obtained in step (d) to additional rounds of selection by repeating steps (c) and (d),
f. recovering cells that have survived at least three rounds of selection obtained in step (e),
g. comparing the selected population of cells obtained in step (f) to the starting population of cells under selection conditions to confirm the enhanced survival of selected cells,
h. plating out the selected pool of cells obtained in step (g) to get isolated colonies,
i. isolating DNA from individual yeast colonies obtained in step (h) and transforming into E. coli to recover the plasmid present in individual yeast colonies,
j. transforming the plasmids obtained in step (i) into yeast and testing the transformants to check if the plasmids really confer enhanced tolerance under the selection conditions, and,
k. identifying the genes carried on the plasmids obtained in step (j), which confer enhanced stress tolerance, by sequencing.

2. A method for identifying genes that increase the stress tolerance of yeast as claimed in claim 1, wherein under fermentation conditions the method comprising of the following steps:
a. transforming yeast with a library of yeast genes cloned in a plasmid to provide a large number of yeast transformants.
b. pooling the transformants obtained in step (a) to provide a starting population of transformants.
c. subjecting the population of transformants obtained in step (b) to selection under liquid conditions such that the viability of the population of cells decrease 3 to 200-fold, thereby enriching those transformed cells that can survive better under these conditions,
d. recovering surviving cells obtained in step (c) and growing them in a medium that allows growth of only those cells retaining plasmids,
e. subjecting the cells obtained in step (d) to additional rounds of selection by repeating steps (c) and (d),
f. recovering cells obtained in step (e) that have survived at least one round of selection,
g. isolating total DNA from the starting population of transformants and the selected population obtained in step (f),
h. amplifying specifically only the insert DNA carried on plasmids from the total DNA obtained in step (g) by using plasmid-specifc primers,
i. labeling of insert DNA fragments obtained in step (h) of the starting population of transformants with one fluorescent dye, and that of the selected population with another fluorescent dye.
j. mixing and hybridizing the labeled probes obtained in step (i) to a microarray spotted with DNA corresponding to almost all the genes of yeast.
k. identifying the DNA spots on the microarray obtained in step (j) that show enhanced signal for probe corresponding to the selected population compared to that of starting population, and,
l. isolating recombinant plasmid clones that carry the genes that correspond to the DNA spots obtained in step (k) as those which increase the stress tolerance of yeast during selection.

3. A method as described in claims 1 and 2, wherein the yeast is transformed with a library of genes from organisms that are already tolerant to particular stress.

4. A method as described in claims 1 to 3, wherein the yeast strain used is *S.cerevisiae.*

5. A method as described in claims 1 to 4, wherein the plasmid is an expression plasmid with a constitutive promoter.

6. A method as described in claims 1 to 4, wherein the plasmid is an expression plasmid with an inducible promoter.

7. A method as described in claims 1 to 6, wherein the plasmid is an integrating plasmid.

8. A method as described in claims 1 to 7, wherein the genes are selected from a group consisting of RPII, WSC2, WSC4, YIL055C, SRA1, ECM39, SSK2, MK71, SOL1 and ADE16.

## Patentansprüche

1. Verfahren zum Identifizieren von Genen zum Erhöhen der Stresstoleranz von Hefe, wobei das genannte Verfahren die folgenden Schritte beinhaltet:
a. Transformieren von Hefe mit einer Bibliothek von Hefegenen, die in einem Plasmid kloniert sind, um eine große Zahl von Hefetransformanten zu erzeugen,
b. Zusammenfassen der in Schritt (a) erhaltenen Transformanten, um eine Ausgangspopulation von Transformanten bereitzustellen,
c. Aussetzen der in Schritt (b) erhaltenen Transformantenpopulation einer Selektion unter Flüssigkeitsbedingungen, so dass die Lebensfähigkeit der Zellpopulation um das 3- bis 200fache abnimmt, wodurch solche transformierten Zellen angereichert werden, die unter diesen Bedingungen besser überleben können,
d. Gewinnen überlebender Zellen, die in Schritt (c) erhalten wurden, und Kultivieren dieser Zellen in einem Medium, das das Wachstum von nur solchen Zellen ermöglicht, die Plasmide bewahren,
e. Aussetzen der in Schritt (d) erhaltenen Zellen zusätzlichen Selektionsrunden durch Wiederholen der Schritte (c) und (d),
f. Gewinnen von in Schritt (e) erhaltenen Zellen, die wenigstens drei Selektionsrunden überlebt haben,
g. Vergleichen der in Schritt (f) erhaltenen selektierten Zellpopulation mit der Ausgangszellpopulation unter Selektionsbedingungen, um das verbesserte Überleben selektierter Zellen zu bestätigen,
h. Ausplattieren des in Schritt (g) erhaltenen selektierten Zellpools, um isolierte Kolonien zu erhalten,
i. Isolieren von DNA von individuellen Hefekolonien, die in Schritt (h) erhalten wurden, und Transformieren in E. coli, um das in individuellen Hefekolonien vorhandene Plasmid zu gewinnen,
j. Transformieren der in Schritt (i) erhaltenen Plasmide in Hefe und Testen der Transformanten, um zu prüfen, ob die Plasmide wirklich eine verbesserte Toleranz unter den Selektionsbedingungen verleihen, und
k. Identifizieren der auf den in Schritt (j) erhaltenen Plasmiden getragenen Gene, die eine verbesserte Stresstoleranz verleihen, durch Sequenzierung.

2. Verfahren zum Identifizieren von die Stresstoleranz von Hefe erhöhenden Genen nach Anspruch 1, wobei das Verfahren unter Fermentationsbedingungen die folgenden Schritte beinhaltet:
a. Transformieren von Hefe mit einer Bibliothek von Hefegenen, die in einem Plasmid kloniert sind, um eine große Zahl von Hefetransformanten zu erzeugen,
b. Zusammenfassen der in Schritt (a) erhaltenen Transformanten, um eine Ausgangspopulation von Transformanten bereitzustellen,
c. Aussetzen der in Schritt (b) erhaltenen Transformantenpopulation einer Selektion unter Flüssigkeitsbedingungen, so dass die Lebensfähigkeit der Zellpopulation um das 3- bis 200fache abnimmt, wodurch solche transformierten Zellen angereichert werden, die unter diesen Bedingungen besser überleben können,
d. Gewinnen überlebender Zellen, die in Schritt (c) erhalten wurden, und Kultivieren dieser Zellen in einem Medium, das das Wachstum von nur solchen Zellen ermöglicht, die Plasmide bewahren,
e. Aussetzen der in Schritt (d) erhaltenen Zellen zusätzlichen Selektionsrunden durch Wiederholen der Schritte (c) und (d),
f. Gewinnen von in Schritt (e) erhaltenen Zellen, die wenigstens eine Selektionsrunde überlebt haben,
g. Isolieren von Gesamt-DNA von der Ausgangspopulation von Transformanten und der in Schritt (f) erhaltenen selektierten Population,
h. Amplifizieren von speziell nur der Insert-DNA, die auf Plasmiden von der in Schritt (g) erhaltenen Gesamt-DNA getragen wird, unter Verwendung von plasmidspezifischen Primern,
i. Markieren von in Schritt (h) erhaltenen Insert-DNA-Fragmenten von der Ausgangspopulation von Transformanten mit einem fluoreszierenden Farbstoff und von solchen der selektierten Population mit einem anderen fluoreszierenden Farbstoff,
j. Mischen und Hybridisieren der in Schritt (i) erhaltenen markierten Sonden mit einem Mikroarray, das mit DNA bedruckt ist, die fast allen Genen von Hefe entspricht,
k. Identifizieren der DNA-Spots auf dem in Schritt (j) erhaltenen Mikroarray, die für eine Sonde, die der selektierten Population entspricht, ein verstärktes Signal im Vergleich zu dem der Ausgangspopulation aufweisen, und
l. Isolieren von rekombinanten Plasmidklonen, die die Gene tragen, die den in Schritt (k) erhaltenen DNA-Spots entsprechen, als solche, die die Stresstoleranz von Hefe während der Selektion erhöhen.

3. Verfahren nach Anspruch 1 und 2, bei dem die Hefe mit einer Bibliothek von Genen von Organismen transformiert wird, die gegenüber speziellem Stress bereits tolerant sind.

4. Verfahren nach den Ansprüchen 1 bis 3, bei dem der verwendete Hefestamm *S. cerevisiae* ist.

5. Verfahren nach den Ansprüchen 1 bis 4, bei dem das Plasmid ein Expressionsplasmid mit einem konstitutiven Promotor ist.

6. Verfahren nach den Ansprüchen 1 bis 4, bei dem das Plasmid ein Expressionsplasmid mit einem induzierbaren Promotor ist.

7. Verfahren nach den Ansprüchen 1 bis 6, bei dem das Plasmid ein integrierendes Plasmid ist.

8. Verfahren nach den Ansprüchen 1 bis 7, wobei die Gene ausgewählt werden aus einer Gruppe bestehend aus RPI1, WSC2, WSC4, YIL055C, SRA1, ECM39, SSK2, MK71, SOL1 und ADE16.

## Revendications

1. Procédé d'identification de gènes qui augmentent la tolérance au stress de la levure dans lequel ledit procédé comprend :
a. la transformation de la levure avec une banque de gènes de levure clonés dans un plasmide afin de fournir un grand nombre de transformants de levure,
b. le regroupement des transformants obtenus à l'étape (a) afin de fournir une population de transformants de départ,
c. la soumission de la population de transformants obtenue à l'étape (b) à une sélection dans des conditions liquides de telle sorte que la viabilité de la population de cellules chute de 3 à 200 fois, enrichissant ainsi les cellules transformées qui peuvent mieux survivre dans ces conditions,
d. la récupération des cellules survivantes obtenues à l'étape (c) et leur culture dans un milieu qui permet la croissance uniquement des cellules qui retiennent les plasmides,
e. la soumission des cellules obtenues à l'étape (d) à d'autres cycles de sélection en répétant les étapes (c) et (d),
f. la récupération des cellules qui ont survécu à au moins trois cycles de sélection obtenues à l'étape (e),
g. la comparaison de la population sélectionné de cellules obtenues à l'étape (f) à la population de cellules de départ dans les conditions de sélection afin de confirmer la survie rehaussée des cellules sélectionnées,
h. le dépôt du groupe sélectionné de cellules obtenues à l'étape (g) afin d'obtenir des colonies isolées,
i. l'isolation de l'A.D.N. des colonies de levure individuelles obtenues à l'étape (h) et la transformation en E.coli afin de récupérer le plasmide présent dans les colonies de levure individuelles,
j. la transformation en levure des plasmides obtenus à l'étape (i) et l'essai des transformants afin de vérifier si les plasmides confèrent réellement une tolérance rehaussée dans les conditions de sélection, et,
k. l'identification des gènes portés sur les plasmides obtenus à l'étape (j), lesquels confèrent une tolérance rehaussée au stress, par séquençage.

2. Procédé d'identification de gènes qui augmentent la tolérance au stress de la levure selon la revendication 1, dans lequel dans des conditions de fermentation le procédé comprend les étapes suivantes :
a. la transformation de la levure avec une banque de gènes de levure clonés dans un plasmide afin de fournir un grand nombre de transformants de levure,
b. le regroupement des transformants obtenus à l'étape (a) afin de fournir une population de transformants de départ,
c. la soumission de la population de transformants obtenue à l'étape (b) à une sélection dans des conditions liquides de telle sorte que la viabilité de la population de cellules chute de 3 à 200 fois, enrichissant ainsi les cellules transformées qui peuvent mieux survivre dans ces conditions,
d. la récupération des cellules survivantes obtenues à l'étape (c) et leur culture dans un milieu qui permet la croissance uniquement des cellules qui retiennent les plasmides,
e. la soumission des cellules obtenues à l'étape (d) à d'autres cycles de sélection en répétant les étapes (c) et (d),
f. la récupération des cellules obtenues à l'étape (e) qui ont survécu à au moins un cycle de sélection,
g. l'isolation de l'A.D.N. total de la population de transformants de départ et de la population sélectionnée obtenue à l'étape (f),;
h. l'amplification spécifiquement et uniquement de l'A.D.N. d'insert porté sur les plasmides de l'A.D.N. total obtenu à l'étape (g) en utilisant des amorces spécifiques aux plasmides,
i. l'étiquetage de fragments d'A.D.N. d'insert obtenus à l'étape (h) de la population de transformants de départ avec un colorant fluorescent, et de celui de la population sélectionnée avec un autre colorant fluorescent,
j. le mixage et l'hybridation des sondes étiquetées obtenues à l'étape (i) en une micro-matrice à points d'A.D.N. correspondant à presque tous les gènes de levure,
k. l'identification des points d'A.D.N. sur la micro-matrice obtenue à l'étape (j) qui montrent un signal rehaussé pour la sonde correspondant à la population sélectionnée en comparaison à celui de la population de départ, et,
l. l'isolation des clones à plasmides recombinants qui portent des gènes qui correspondent aux points d'A.D.N. obtenus à l'étape (k) comme ceux qui augmentent la tolérance au stress de la levure durant la sélection.

3. Procédé selon les revendications 1 et 2, dans lequel la levure est transformée avec une banque de gènes provenant d'organismes qui sont déjà tolérants à un stress particulier.

4. Procédé selon les revendications 1 à 3, dans lequel la souche de levure utilisée est *S.cerevisiae.*

5. Procédé selon les revendications 1 à 4, dans lequel le plasmide est un plasmide d'expression à promoteur constitutif.

6. Procédé selon les revendications 1 à 4, dans lequel le plasmide est un plasmide d'expression à promoteur inductible.

7. Procédé selon les revendications 1 à 6, dans lequel le plasmide est un plasmide d'intégration.

8. Procédé selon les revendications 1 à 7, dans lequel les gènes sont sélectionnés dans un groupe consistant en RPI 1, WSC 2, WSC 4, YIL 055C, SRA 1, ECM 39, SSK 2, MK7 1, SOL 1 et ADE 16.
